# FASCICULE DE BREVET EUROPEEN

(11) **EP 0 808 332 B1**
(45) Date de publication et mention de la délivrance du brevet: **24.05.2000**
(21) Numéro de dépôt: 95904572.5
(22) Date de dépôt: 20.12.1994
(51) Int. Cl.: C07K 14/78, A61K 7/48

(54) **PROCEDE DE PREPARATION DE COLLAGENE A PARTIR DE CNIDAIRES, ET COMPOSITIONS OBTENUES UTILISABLES EN COSMETIQUE**
METHODE ZUR HERSTELLUNG VON KOLLAGEN AUS CNIDARIANS UND ENTSPRECHENDE KOSMETISCHE ZUSAMMENSETZUNGEN
METHOD FOR PREPARING COLLAGEN FROM CNIDARIANS, AND RESULTING COSMETIC COMPOSITIONS

(30) Priorité: 20.12.1993 FR 9315318
(43) Date de publication de la demande: 26.11.1997
(73) Titulaire: JAVENECH (Société Anonyme), 35133 Fougères (FR)
(72) Inventeur: RANSON, Michèle, F-75017 Paris (FR); DAVID, Marc, F-35300 Fougères (FR)
(74) Mandataire: L'Helgoualch, Jean
(86) Numéro de dépôt international: FR9401494
(87) Numéro de publication internationale: WO9517428

(56) Documents cités:
- EP-A- 0 419 111
- FR-A- 2 678 624
- JOURNAL OF BIOLOGICAL CHEMISTRY, vol. 260, BALTIMORE, MD US, pages 15352-15356, S. MIURA AND S. KIMURA 'Jellyfish Mesogloea Collagen'
- JOURNAL OF FOOD SCIENCE, vol. 48, CHICAGO US, pages 1758-1760, S. KIMURA ET AL. 'Collagen as the Major Edible Component of Jellyfish (Stomolophus nomural)'
- DATABASE WPI Section Ch, Week 2390 Derwent Publications Ltd., London, GB; Class D21, AN 90-175248 & JP,A,02 115 112 (POLA CHEM IND KK) , 27 Avril 1990

## Description

La présente invention concerne un collagène présentant une composition déterminée en acides aminés, un procédé de préparation de collagène d'origine marine ayant cette composition, et plus particulièrement un procédé de préparation de collagène à partir de cnidaires, notamment de méduses, ainsi que des compositions cosmétiques le contenant.

Le collagène est une substance protéinique dont les propriétés sont mises à profit dans divers domaines techniques notamment l'industrie du papier, du textile, des produits alimentaires, des produits pharmaceutiques et des produits cosmétiques. Il est généralement obtenu à partir de substances animales telles que des peaux de bovins ou des peaux de poissons.

Ainsi, le brevet FR-A-2.678.624 décrit un procédé de préparation de collagène utilisable dans diverses industries, consistant à utiliser la peau non pigmentée de certains poissons plats, pour extraire le collagène à l'état natif et le séparer par précipitation à partir du surnageant. Le brevet FR-A-2.636.333 concerne un procédé de préparation de collagène à partir de peaux fraîches, notamment de bovins, suivant lequel on lave les peaux puis on les traite par voie mécanique pour défibriller le tissu dermique. Le brevet EP-A-079.398 décrit la préparation de collagène à usage chirurgical par traitement de peaux par voie chimique, consistant à effectuer une désagrégation alcaline suivie d'un traitement à l'acide chlorhydrique, d'un lavage et d'une réticulation des fibres collagéniques en présence d'un agent émulsifiant.

L'une des principales difficultés inhérentes aux procédés connus résulte du grand nombre d'impuretés, contaminants et agents non conventionnels provenant des diverses sources utilisées, telles que peaux de bovins ou de poissons, et quand le collagène doit être utilisé dans l'industrie des produits pharmaceutiques ou cosmétiques, il est indispensable qu'il soit extrêmement propre, et en conséquence il est alors nécessaire d'effectuer de nombreuses opérations supplémentaires de purification, qui alourdissent considérablement le coût économique de la préparation. Ainsi, dans le cas du collagène d'origine bovine, le mode de préparation doit tenir compte des risques de contamination virale ou d'agents non conventionnels (prions), tandis que dans le cas du collagène de poissons, on utilise généralement des déchets ou des sousproduits de récupération qui peuvent contenir des contaminants. De plus, la composition du collagène en acides aminés varie sensiblement en fonction de la source utilisée et du procédé de préparation.

On sait que les cnidaires sont des organismes relativement simples, du type métazoaire, contenant essentiellement de l'eau et du collagène, dont les méduses sont la phase principale. La composition en acides aminés du collagène des méduses a été analysée [voir par exemple S. Kimura et al., "Le collagène comme principal constituant comestible de la méduse", J. of Food Science, vol.48, pp.1758-1760 (1983)], mais l'utilisation du collagène de cnidaires, ou de méduses, dans des compositions cosmétiques n'a jamais été décrite, sans doute en raison des propriétés urticantes bien connues de nombreuses espèces.

S. Miura et al. (J. of Biological Chemistry, vol. 260, p.15352-15356) décrivent un collagène extrait de mésoglée, obtenu par extraction acide suivie d'une digestion par la pepsine.

Le brevet JP-A-2115112 décrit également la préparation de collagène de méduse par extraction acide et addition de pepsine suivie d'un ajustement du pH à 8 pour précipiter les fibres de collagène.

On a donc été amené à rechercher de nouveaux procédés de préparation de collagène ou de nouvelles sources de collagène évitant les inconvénients précités, c'est-à-dire permettant d'obtenir du collagène exempt de contaminants divers et présentant une pureté suffisante pour une utilisation dans l'industrie pharmaceutique et cosmétique, avec un rendement satisfaisant.

Or, on a constaté, à la suite des travaux réalisés par les inventeurs, qu'il est possible d'obtenir du collagène natif de la matrice extra-cellulaire et de composition adaptée à une utilisation en cosmétique, en effectuant une extraction à partir de cnidaires.

La présente invention a donc pour objet un procédé de préparation de collagène utilisable dans l'industrie des produits cosmétiques, à partir de cnidaires, permettant d'obtenir un collagène de bonne qualité et d'excellente pureté.

L'invention a également pour objet le collagène obtenu par ledit procédé, présentant une composition déterminée en acides aminés, ainsi que des compositions cosmétiques le contenant.

Suivant la présente invention, le procédé de préparation de collagène utilisable dans des compositions pharmaceutiques et cosmétiques, consiste à effectuer un lavage de cnidaires, suivi d'une extraction acide, d'une centrifugation, et d'une précipitation saline.

Il est préférable de traiter des cnidaires, notamment des méduses de la classe des schyphoméduses, préalablement congelées, que l'on broie avant et/ou après l^{'}étape de lavage. Suivant une forme préférentielle de réalisation de l'invention, on traite des schyphoméduses de l'ordre Rhizostoma, plus particulièrement Rhizostoma pulmo.

L'étape d'extraction acide peut s'effectuer par exemple en milieu acide acétique de concentration comprise entre 0,2 M et 2 M, suffisante pour solubiliser le collagène.

L'étape de précipitation saline, après centrifugation suivant une technique classique, peut s'effectuer par exemple au moyen d'une solution aqueuse de chlorure de sodium, en concentration appropriée, de telle sorte que la force ionique du milieu soit comprise entre 1 et 4.

Il est préférable, conformément à l'invention, que la température ne dépasse pas 15°C environ dans les extracteurs, afin d'éviter une dénaturation du collagène.

Le procédé conforme a la présente invention présente l'avantage d'être utilisable directement à l'échelle industrielle. Il permet de traiter simplement, par lots ou en continu, des quantités importantes de méduses, pouvant aller de quelques dizaines à plusieurs centaines de kilogrammes, et procure un collagène natif de poids moléculaire important, ne comportant pratiquement pas de sous-unités inférieures à 100.000 Daltons. Ainsi, à partir d'environ 600 kg de méduses, on peut préparer près de 2 kg de fibres essorées de collagène comprenant environ 5% de protéines collagéniques.

De plus, le procédé de l'invention utilise des cnidaires entiers, notamment des méduses, et non des sous-produits ou des fragments d'organismes.

Le collagéne obtenu conformément à la présente invention se distingue en ce que sa composition en acides aminés comprend moins de 9% d'alanine et au moins 0,5% de cystine. De préférence, la teneur en alanine est comprise entre 3% et 8%, et la teneur en cystine est comprise entre 0,8% et 1,2%.

On constate en outre un taux d'hydroxyproline réduit de moitié environ par rapport aux extraits collagéniques de poissons connus dans la technique, et un taux d'hydroxylysine élevé, supérieur à 2% et pouvant atteindre 4 à 6%, entraînent une augmentation des liaisons interchaînes et une amélioration des propriétés rhéologiques des solutions obtenues à partir de ce collagène. Enfin, le taux d'arginine est nettement supérieur au taux observé dans les collagènes de poissons, ce qui contribue à améliorer la stabilité du produit, notamment vis-à-vis de l'oxydation, des effets des radicaux libres, et des ultraviolets.

Un des avantages de l'invention est que l'on obtient un collagène natif de la matrice extracellulaire qu'il n'est pas nécessaire de traiter par hydrolyse enzymatique, contrairement aux collagènes usuels de la technique qu'il faut hydrolyser par addition de pepsine pour améliorer leur solubilité.

La température de début de dénaturation du collagène de méduses conforme à l'invention est voisine de 27-28°C, tandis que la température de dénaturation des collagènes acido-solubles de mammifères est de l'ordre de 38°C. Cette température plus basse constitue une caractéristique avantageuse du collagène de l'invention.

Le type de collagène a été déterminé par une technique immuno-enzymatique (ELISA) fondée sur l'utilisation d'anticorps dirigés contre les différents type connus de collagène (collagène de types I, III, IV et V). Après sensibilisation des plaques, réaction avec les anticorps et révélation suivant la technique usuelle, le type de collagène est identifié par mesure de la densité optique.

Les essais de tolérance effectués avec le collagène de l'invention suivant une méthode usuelle, ont montré un indice d'irritation cutanée primaire inférieur à 0,4, significatif de l'absence d'irritation cutanée. De même, les essais d'irritation oculaire ont montré que le collagène de l'invention est très faiblement irritant (indice d'irritation maximale à 1 heure égal à 7,6).

Les compositions cosmétiques préparées conformément à la présente invention ont fait apparaître d'intéressantes propriétés, notamment en ce qui concerne l'effet de stimulation de la cicatrisation des plaies, de régénération des tissus, d'assouplissement et d'adoucissement de la peau, lorsque la composition est appliquée sur la peau. De plus, le collagène présente des propriétés filmogènes et hémostatiques.

Les compositions à base de collagène suivant l'invention peuvent être présentées sous les formes usuelles de crème, émulsions, pommades ou gels.

Les exemples suivants, donnés à titre non limitatifs, décrivent la préparation de collagène conformément à l'invention, ainsi que des compositions adaptées à une utilisation en cosmétique.

### Exemple 1

On concasse 600 kg de méduses congelées, que l'on laisse reposer en chambre froide (4°C) jusqu'à décongélation complète.

Après lavage à l'eau, on procède à un broyage puis à une extraction en milieu acétique (acide acétique 0,5 M) dans un extracteur de 5 m³. L'extraction est réalisée pendant environ 12 heures, sous faible agitation au moyen d'un agitateur rotatif (50 trs/min) à une température comprise entre 12 et 15°C.

L'extrait est ensuite centrifugé en continu sur une centrifugeuse industrielle, et après séparation des particules en suspension, le centrifugat est précipité par addition de chlorure de sodium.

Les fibres de protéines collagéniques précipitées en milieu salin (force ionique 2) sont essorées pour obtenir environ 2 kg de fibres à 5% de protéines collagéniques.

Des solutions aqueuses dosées à 0,1%, 0,3% ou 0,6% de protéines collagéniques sont préparées à partir des fibres.

On obtient ainsi un extrait collagénique se présentant sous forme de liquide visqueux légèrement opalescent, miscible à l'eau, de pH voisin de 4,0.

La composition en acides aminés est la suivante, comparée à celle d'un extrait collagénique de type connu, obtenu à partir de peaux de poissons:

| Acide aminé | Collagène de l'invention | collagène de peaux de poissons |
|---|---|---|
| Acide aspartique | 7,08 | 5,65 |
| Thréonine | 3,29 | 2,04 |
| Sérine | 3,89 | 2,38 |
| Acide glutamique | 11,63 | 8,51 |
| Proline | 11,12 | 11,91 |
| Glycine | 31,34 | 32,67 |
| Alanine | 4,45 | 12,59 |
| Valine | 2,63 | 2,79 |
| Cystine | 1,01 | 0,00 |
| Méthionine | 0,16 | 0,20 |
| Isoleucine | 2,17 | 1,29 |
| Leucine | 3,13 | 2,93 |
| Tyrosine | 0,49 | 0,00 |
| Phénylalanine | 1,31 | 1,70 |
| Histidine | 0,86 | 0,88 |
| Lysine | 3,28 | 3,40 |
| Arginine | 6,57 | 4,70 |
| Hydroxyproline | 3,03 | 5,92 |
| Hydroxylysine | 3,04 | 0,41 |

L'analyse de la composition en acides aminés a été effectuée par chromatographie sur colonne échangeuse d'ions de la protéine hydrolysée, suivant une technique classique.

Les résultats de l'analyse indiqués ci-dessus montrent que le collagène obtenu suivant la présente invention contient environ 1% de cystine tandis que le collagène de type connu n'en contient pas. De plus, sa teneur en alanine (4,45%) est nettement réduite par rapport à celle du collagène connu (12,59%).

La présence de cystine est particulièrement intéressante, conformément à l'invention, car elle améliore considérablement la qualité cosmétologique des compositions cosmétiques préparées avec le collagène de l'invention en raison de l'affinité de la cystine pour la kératine de la peau.

D'autre part, on constate que le taux d'hydroxylysine est supérieur à 3% (contre moins de 0,5% pour le collagène de peau de poisson), ce qui améliore sensiblement les propriétés rhéologiques des solutions obtenues à partir de ce collagène matriciel.

### Exemple 2

On prépare une émulsion huile dans eau en mélangeant les composants suivants, suivant la technique usuelle :

| | |
|---|---|
| Propylène glycol | 2,0 g |
| PEG 400 | 3,0 g |
| Conservateur | 0,3 g |
| Carbopol 941 | 0,2 g |
| Myristate d'isopropyle | 1,0 g |
| Alcool cétylique | 3,0 g |
| Acide stéarique | 3,0 g |
| Huile de germe de maïs | 2,0 g |
| Parfum | 0,5 g |
| Collagène de l'invention (à 0,6%) | 4,0 g |
| Eau déminéralisée, quantité suffisante pour | 100,0 g |

On utilise du collagène en solution aqueuse à 0,6%, contenant 6 g de collagène par litre de liquide.

### Exemple 3

On prépare une émulsion eau dans huile en mélangeant les composants indiqués ci-après, suivant la technique usuelle :

| | |
|---|---|
| Protegin | 19,0 g |
| Huile de vaseline | 11,0 g |
| Glycérine | 4,0 g |
| Sulfate de magnésium | 0,5 g |
| Parfum | 0,6 g |
| Conservateur | 0,2 g |
| Collagène de l'invention (à 0,6%) | 2,0 g |

On utilise du collagène en solution aqueuse à 0,6%, contenant 6 g de collagène par litre de liquide.

### Exemple 4

On prépare un gel hydro-alcoolique en mélangeant les composants suivants, suivant la technique usuelle :

| | |
|---|---|
| Carbopol 941 | 1,0 g |
| Triéthanolamine | 1,0 g |
| Ethanol à 95% | 60,0 g |
| Glycérol | 3,0 g |
| Propylène glycol | 1,0 g |
| Collagène de l'invention (à 0,6%) | 1,0 g |
| Eau, quantité suffisante pour | 100,0 g |

## Revendications

1. Procédé de préparation de collagène de composition en acides aminés comprenant entre 3% et 8% d'alanine et au moins 0,5% de cystine utilisable dans des compositions pharmaceutiques et cosmétiques, caractérisé en ce qu'il consiste à effectuer un lavage de cnidaires, suivi d'une extraction acide, d'une centrifugation, et d'une précipitation saline.

2. Procédé selon la revendication 1, caractérisé en ce que l'on traite des cnidaires, notamment des méduses de la classe des schyphoméduses, préalablement congelées, que l'on broie avant et/ou après l'étape de lavage.

3. Procédé selon l'une quelconque des revendications 1 et 2, caractérisé en ce que l'on traite des cnidaires constitués par Rhizostoma pulmo.

4. Procédé selon la revendication 1, caractérisé en ce que l'extraction acide est réalisée en milieu acide acétique de concentration comprise entre 0,2 M et 2 M.

5. Procédé selon la revendication 1, caractérisé en ce que la précipitation saline est réalisée au moyen d'une solution aqueuse de chlorure de sodium, en concentration telle que la force ionique du milieu soit comprise entre 1 et 4.

6. Collagène extrait de cnidaires utilisable dans des compositions pharmaceutiques et cosmétiques, caractérisé en ce que sa composition en acides aminés comprend entre 3% et 8% d'alanine et au moins 0,5% de cystine.

7. Collagène selon la revendication 6, caractérisé en ce que sa teneur en cystine est comprise entre 0,8% et 1,2%.

8. Collagène selon l'une quelconque des revendications 6 et 7, caractérisé en ce que sa composition en hydroxylysine est supérieure à 2%.

9. Composition cosmétique caractérisée en ce qu'elle contient du collagène selon l'une quelconque des revendications 6 à 8, associé à un support acceptable en cosmétique.

10. Composition cosmétique selon la revendication 9, caractérisée en ce qu'elle est sous forme de crème, de gel, d'émulsion ou de pommade.

## Claims

1. A process for preparing collagen of an amino acid composition comprising between 3% and 8% alanine and at least 0.5% cystine, usable in pharmaceutical and cosmetic compositions, characterised in that it consists in washing cnidarians, followed by acid extraction, centrifuging and saline precipitation.

2. A process according to Claim 1, characterised in that cnidarians, in particular pre-frozen jellyfish of the scyphomedusa class, which are crushed before and/or after the washing stage, are treated.

3. A process according to any one of Claims 1 and 2, characterised in that cnidarians consisting of Rhizostoma pulmo are treated.

4. A process according to Claim 1, characterised in that the acid extraction is effected in an acetic acid medium of a concentration of between 0.2 M and 2 M.

5. A process according to Claim 1, characterised in that the saline precipitation is effected by means of an aqueous sodium chloride solution, in a concentration such that the ionic strength of the medium is between 1 and 4.

6. Collagen extracted from cnidarians which can be used in pharmaceutical and cosmetic compositions, characterised in that its amino acid composition comprises between 3% and 8% alanine and at least 0.5% cystine.

7. Collagen according to Claim 6, characterised in that its cystine content is between 0.8% and 1.2%.

8. Collagen according to any one of Claims 6 and 7, characterised in that its hydroxylysine composition is greater than 2%.

9. A cosmetic composition, characterised in that it contains collagen according to any one of Claims 6 to 8, associated with a cosmetically acceptable carrier.

10. A cosmetic composition according to Claim 9, characterised in that it is in the form of a cream, a gel, an emulsion or a pomade.

## Patentansprüche

1. Verfahren zur Herstellung von Kollagen mit einer Aminosäure-Zusammensetzung enthaltend zwischen 3 und 8 % Alanin und zumindest 0,5 % Cystein, das in pharmazeutischen und kosmetischen Zusammensetzungen einsetzbar ist, dadurch gekennzeichnet, dass es darin besteht, eine Waschung von Nesseltieren (Cnidaria), gefolgt von Säureextraktion, Zentrifugation und Aussalzung durchzuführen.

2. Verfahren nach Anspruch 1, dadurch gekennzeichnet, dass Nesseltiere, insbesondere Medusen der Klasse der Scyphomedusen, vorzugsweise tiefgefroren, behandelt werden, die vor und/oder nach dem Waschschritt fein zermahlen werden.

3. Verfahren nach einem der Ansprüche 1 oder 2, dadurch gekennzeichnet, dass Nesseltiere der Spezies Rhizostomea pulmo behandelt werden.

4. Verfahren nach Anspruch 1, dadurch gekennzeichnet, dass die Säureextraktion in Essigsäuremedium mit einer Konzentration zwischen 0,2 M und 2 M durchgeführt wird.

5. Verfahren nach Anspruch 1, dadurch gekennzeichnet, dass die Aussalzung mit einer wässrigen Natriumchloridlösung mit einer solchen Konzentration durchgeführt wird, dass die lonenstärke des Mediums zwischen 1 und 4 liegt.

6. Nesseltier-Kollagenextrakt, der in pharmazeutischen und kosmetischen Zusammensetzungen einsetzbar ist, dadurch gekennzeichnet, dass seine Aminosäure-Zusammensetzung zwischen 3 und 8 % Alanin und zumindest 0,5 % Cystein enthält.

7. Kollagen nach Anspruch 6, dadurch gekennzeichnet, dass sein Cysteingehalt zwischen 0,8 % und 1,2 % liegt.

8. Kollagen nach einem der Ansprüche 6 und 7, dadurch gekennzeichnet, dass sein Hydroxylysingehalt über 2 % liegt.

9. Kosmetische Zusammensetzung, dadurch gekennzeichnet, dass sie Kollagen nach einem der Ansprüche 6 bis 8 zusammen mit einem kosmetisch verträglichen Träger enthält.

10. Kosmetische Zusammensetzung nach Anspruch 9, dadurch gekennzeichnet, dass sie in Form einer Creme, eines Gels, einer Emulsions oder einer Pomade vorliegt.
